# DEMANDE DE BREVET EUROPEEN

(11) **EP 4 718 069 A1**
(43) Date de publication de la demande: **01.04.2026**
(21) Numéro de dépôt: 25204116.5
(22) Date de dépôt: 23.09.2025
(51) Int. Cl.: G01N 33/493, A61B 5/145, A61B 5/20, A61B 5/00, A61B 10/00

(54) **CARTOUCHE POUR ANALYSE D'URINE**

(30) Priorité: 27.09.2024 FR 2410350
(71) Demandeur: Withings, 92130 Issy-les-Moulineaux (FR)
(72) Inventeur: Geffraye, Victor, 92130 Issy-les-Moulineaux (FR); Donnet, Pierre-Arnaud, 92130 Issy-les-Moulineaux (FR)
(74) Mandataire: Withings IP

(57) **Abrégé**

Il est proposé une cartouche (202) pour analyse d'urine comprenant un support (300) et une pluralité de chambres (310) étanches, chaque chambre (310) étant propre à recevoir de l'urine pour analyse.

La pluralité de chambres (310) étanches est maintenue en position par le support (300). Au moins une portion du support, appelé portion dessiccante (420), est composée d'un dessiccant solide, apte à absorber l'humidité de l'air au contact de la portion dessiccante (420), la portion dessiccante (420) étant à l'extérieur des chambres (310) étanches.

## Description

La présente invention concerne une cartouche pour analyse d'urine. L'invention concerne également un dispositif d'analyse d'urine comprenant une telle cartouche et une station configurée pour être positionnée sur une paroi d'une cuvette de toilettes.

### Etat de la technique

De nombreux paramètres biologiques sont reflétés dans l'urine d'un individu. A partir d'un échantillon d'urine, il est par exemple possible de détecter des problèmes de santé tels qu'une infection urinaire, le diabète ou une insuffisance rénale. L'échantillon d'urine peut également refléter la qualité d'une alimentation, identifier une période de fécondité ou une grossesse et détecter une prise de drogues ou de tabac. Il est alors intéressant de surveiller divers paramètres biologiques périodiquement.

Le document WO2021/175909 décrit un dispositif qui se loge sur une paroi d'une cuvette de toilettes et recueille un échantillon d'urine avant d'effectuer une analyse optique. Le dispositif comprend une station et une cartouche qui peut être retirée et remplacée de la station. La cartouche contient des réactifs réagissant avec l'urine, notamment des bandelettes de test urinaires. Ces réactifs sont disposés dans des chambres respectives. Le document EP4338839 décrit notamment une cartouche présentant des logements particulièrement étanches pour préserver la durée de vie des réactifs qui sont sensibles à l'humidité.

Afin de prolonger la durée de vie de réactifs sensibles à l'humidité, il est connu de disposer du dessiccant au contact de ces réactifs. On peut citer par exemple les documents US6497845B1 ou US5575403A qui décrivent du dessiccant disposé dans des logements accueillant du réactif.

### Résumé de l'invention

La présente description se propose d'améliorer encore le fonctionnement et la durée de vie du dispositif d'analyse d'urine en limitant l'humidité dans le dispositif.

A cet effet, la présente description concerne une cartouche pour analyse d'urine comprenant : un support ; une pluralité de chambres étanches, chaque chambre étanche étant propre à recevoir de l'urine pour analyse d'urine; dans laquelle : la pluralité de chambres étanches est maintenue en position par le support ; au moins une portion du support, appelé portion dessiccante, est composée d'un dessiccant solide, apte à absorber l'humidité de l'air au contact de la portion dessiccante, la portion dessiccante étant à l'extérieur des chambres étanches.

En effet, la cartouche peut comprendre plusieurs dizaines de bandelettes de test. Afin d'effectuer une analyse urinaire de l'utilisateur, la station injecte de l'urine dans une chambre. Un analyseur peut alors analyser le résultat de cette réaction et en informer l'utilisateur. Ainsi, à chaque analyse, de l'urine est injectée dans l'une des chambres qui est alors percée. Les inventeurs se sont rendu compte que ces injections répétées augmentent l'humidité dans la station, ce qui peut conduire à un environnement néfaste pour le bon fonctionnement des dispositifs électroniques présents dans la station et la durée de vie du dispositif d'analyse d'urine.

A cet effet, le support de la cartouche selon la présente description offre une double fonction permettant à la fois de supporter mécaniquement les chambres pour les maintenir en position et également d'absorber une partie de l'humidité présente dans la station lorsque la cartouche est en place dans la station. Cette double fonction permet de rendre les parties structurels dessiccante pour maximiser le volume d'absorption. En effet, il existe une contrainte forte de place dans la station ne permettant pas l'ajout d'un volume dessiccant supplémentaire. En outre, la portion dessiccante peut être disposée de sorte qu'elle ne gêne pas l'analyse de l'urine. Enfin, la cartouche étant un élément consommable et remplaçable, le remplacement de la cartouche permet également le remplacement de la portion dessiccante et donc permet de renouveler la capacité d'absorption du dessiccant.

Par chambre étanche, on entend chambre étanche à l'humidité.

Par « à l'extérieur des chambres étanches », on entend que la portion dessiccante n'est pas en contact avec l'intérieur des chambres d'étanches.

Dans un mode de réalisation, chaque chambre étanche loge un réactif configuré pour réagir avec de l'urine.

Dans un mode de réalisation, le dessiccant solide est un matériau hygroscopique.

Dans un mode de réalisation, le dessiccant solide est un mélange bi-matériaux formé d'un polymère plastique et d'un matériau dessiccant.

Dans un mode de réalisation, le dessiccant solide est configuré pour absorber des molécules de vapeur d'eau au contact du support.

Dans un mode de réalisation, le dessiccant solide est formé de polypropylène chargé en zéolite.

Dans un mode de réalisation, la portion dessiccante possède une capacité d'absorption comprise entre 100 mg/g et 300 mg/g, notamment entre 140 mg/g et 200 mg/g.

Dans un mode de réalisation, la portion dessiccante représente au moins 25%, préférentiellement 50%, du support en surface.

Dans un mode de réalisation, la portion dessiccante est rigide.

Dans un mode de réalisation, la portion dessiccante comprend une zone de réception d'effort d'un actionneur d'une station.

Dans un mode de réalisation, la portion dessiccante forme un socle sur lequel sont montées les chambres.

Dans un mode de réalisation, chaque chambre comprend un opercule perçable, par exemple par une seringue.

Dans un mode de réalisation, la zone de réception d'effort est un moyeu et l'actionneur comprend un arbre en rotation.

Dans un mode de réalisation, l'opercule est transparent ou translucide.

Dans un mode de réalisation, chaque réactif est disposé sur une bandelette de test.

Dans un mode de réalisation, le support est un support rotatif.

Dans un mode de réalisation, les chambres sont disposés les unes à côté des autres en forme de cylindre circulaire droit d'au moins 80% d'un cercle.

Dans un mode de réalisation, le cylindre présente un diamètre compris entre 3 et 10 cm.

Dans un mode de réalisation, les chambres sont agencées sur une paroi externe du support rotatif.

Dans un mode de réalisation, chaque chambre s'étend selon une direction principale orthogonale au socle.

Dans un mode de réalisation, la portion dessiccante forme sensiblement un disque.

Dans un mode de réalisation, le disque présente un diamètre compris entre 3 cm et 10 cm.

Dans un mode de réalisation, le disque présente une épaisseur comprise entre 1 mm et 10 mm.

Dans un mode de réalisation, la cartouche comprend en outre un dessiccant additionnel, différent de la portion dessiccante, disposé dans au moins une chambre.

Dans un mode de réalisation, le dessiccant additionnel est une bande de matériau dessiccant.

La présente description concerne également un dispositif d'analyse optique de l'urine comprenant : une cartouche telle que définie ci-dessus ; une station configurée pour être positionnée sur une paroi d'une cuvette de toilettes, la station comprenant : un boitier comprenant un compartiment de réception dans lequel la cartouche est au moins partiellement reçue, un injecteur configuré pour injecter du fluide dans l'une des chambres, et un analyseur pour analyser l'urine injectée dans ladite chambre.

Dans un mode de réalisation, la cartouche est agencée de manière amovible dans le boitier.

Dans un mode de réalisation, l'analyseur comprend une source de lumière et un capteur de lumière, configurés pour émettre et recevoir de la lumière.

Dans un mode de réalisation, l'analyseur est configuré pour analyser un changement de couleur du réactif au contact de l'urine injectée.

Dans un mode de réalisation, l'injecteur est agencé dans une zone qui est, lorsque la cartouche est montée dans la station, radialement interne à la cartouche.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station et la cartouche sont visibles,
- [FIG. 3] : la figure 3 présente une vue détaillée d'une cartouche selon un mode de réalisation en coopération avec un actionneur de la station,
- [FIG. 4] : la figure 4 présente une vue éclatée en perspective de dessous de la cartouche de la figure 3,
- [FIG. 5] : la figure 5 la figure 5 présente une vue éclatée en perspective de dessus de la cartouche de la figure 3,
- [FIG. 6] : la figure 6 présente une vue en coupe d'une cartouche et d'une station selon un mode de réalisation, à l'emplacement d'un analyseur optique de la station,
- [FIG. 7] : la figure 7 présente une vue en perspective d'une cartouche selon un autre mode de réalisation en coopération avec un actionneur de la station, et
- [FIG. 8] : la figure 8 présente une vue en perspective d'une cartouche selon un autre mode de réalisation en coopération avec un actionneur de la station.

### Description détaillée

La présente description présente différents exemples de dispositif d'analyse d'urine comprenant une station telle que divulguée dans les documents WO2021/175909 et WO2021/175944, ci-après dénommés WO'909 et WO'944. Des variantes des stations sont présentées dans les documents WO2023036805, WO2023036806, WO2023036808, WO2023036809, ci-après dénommés WO'80X.

Les paragraphes suivants expliquent le principe général d'un dispositif d'analyse d'urine, mais tous les détails des documents WO'909 et WO'933 (ainsi que de tous les documents PCT susmentionnés) sont applicables.

### Forme générale du boîtier

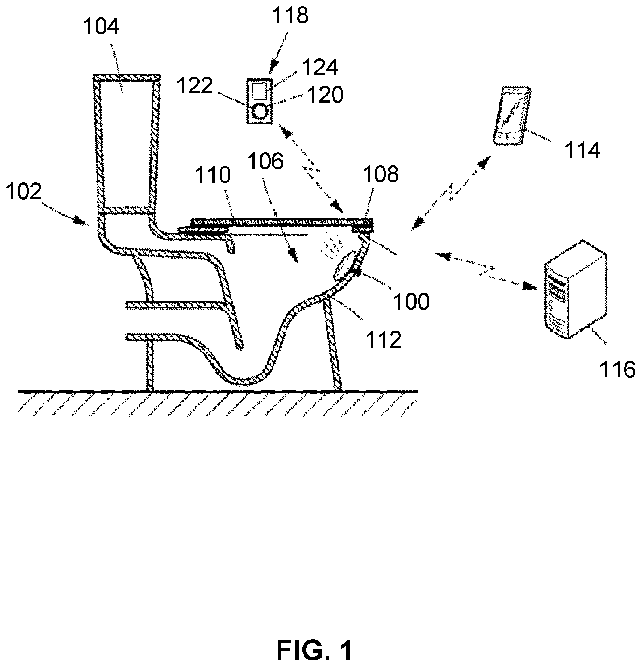

La figure 1 illustre schématiquement un dispositif d'analyse 100 (aussi appelé "dispositif 100" par la suite) pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est configuré pour être placé entièrement dans la cuvette des toilettes. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi interne 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

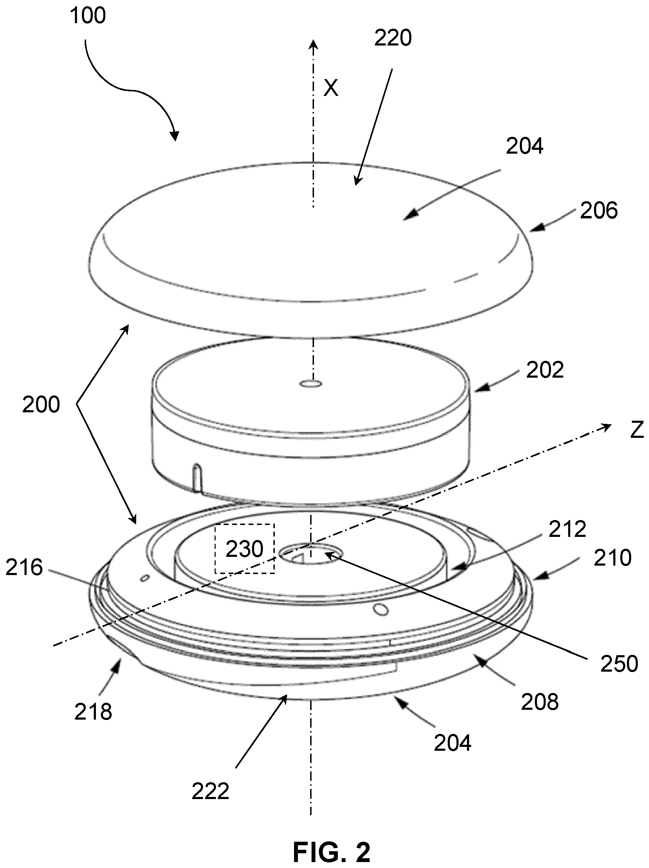

Comme l'illustre plus en détail la figure 2, le dispositif d'analyse 100 peut comprendre une station 200 et une cartouche 202. La cartouche 202 peut être montée de manière amovible sur la station 200. La station 200 peut comprendre un boîtier 204 qui peut comporter deux coques, notamment une coque avant 206 et une coque arrière 208. La coque avant 206 et la coque arrière 208 peuvent coopérer l'une avec l'autre par l'intermédiaire d'un mécanisme de fixation 216, dans un plan normal à l'axe X. La coque avant 206 et la coque arrière 208 peuvent être assemblées de manière réversible, par exemple par vissage ou par clipsage. Dans une variante, la coque avant 206 et la coque arrière 208 peuvent être assemblées de manière permanente, par exemple par collage, clipsage, magnétisation, ou soudage par ultrasons. Bien entendu, d'autres moyens de fixation peuvent être utilisés pour assembler les deux coques.

En particulier, comme illustré sur la figure 2, la coque avant 206 et la coque arrière 208 sont vissées l'une à l'autre. Une partie interne de la coque avant 206 comprend un filetage. Le filetage de la coque avant 206 est destiné à coopérer avec un filetage complémentaire présent sur l'intérieur de la coque arrière 208. Cela permet de démonter facilement le boîtier 204 pour accéder à l'assemblage de test à l'intérieur du boîtier.

Un joint peut être présent entre la coque avant 206 et la coque arrière 208. Ainsi, le boîtier 204 est étanche avec l'extérieur du dispositif 100.

Comme on peut le voir sur les figures, le boîtier 204 peut avoir une forme globale extérieure de galet circulaire. En d'autres termes, le boîtier 204 présente une forme sphéroïde. L'axe X est la ligne centrale du boîtier. Avantageusement, la coque avant 206 peut être sensiblement symétrique en rotation, ce qui donne un aspect aérodynamique au dispositif une fois installé. Le boîtier 204 sert de collecteur d'urine.

Le boîtier 204 comprend une face avant 220 pour recevoir un flux d'urine directement d'un utilisateur urinant sur les toilettes et une face arrière 222 opposée à la face avant 220. Comme illustré à la figure 2, la face avant 220 peut être disposée sur la coque avant 206 et la face arrière 222 peut être disposée sur la coque arrière 208. La face avant 220 est orientée vers l'intérieur de la cuvette 106. La face avant 220 est donc destinée à recevoir l'urine lorsque l'utilisateur urine en s'asseyant sur les toilettes 102. La face arrière 222 fait face à la paroi intérieure 112 de la cuvette 106.

La surface extérieure de la face avant 220 peut être lisse. En d'autres termes, la face avant 220 est dépourvue de crêtes ou de rainures. Ainsi, le flux d'urine entrant en contact avec la face avant 220 s'accroche et s'étale sur la face avant 220. La face avant 220 peut être sensiblement symétrique en rotation autour de l'axe X.

La surface extérieure du boîtier 204 peut également être blanche ou de couleur claire. La couleur de la surface extérieure peut être similaire à celle des toilettes, ce qui accroît la discrétion du dispositif.

Le boîtier 204 peut avoir un diamètre, mesuré dans la direction orthogonale à l'axe X, compris entre 50 mm et 150 mm. Le boîtier 204 peut avoir une épaisseur, mesurée dans la direction de l'axe X, comprise entre 15 mm et 50 mm. Ainsi, le boîtier 204 est suffisamment compact pour être entièrement logé dans la cuvette des toilettes. Le dispositif d'analyse d'urine 100 est discret. De plus, le boîtier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

Selon un autre aspect, dans un mode de réalisation, le boîtier 204 présente un facteur de forme général tel que le rapport entre l'épaisseur et le diamètre est compris entre 0,2 et 0,5, et même de préférence entre 0,3 et 0,4. De telles proportions rappellent un galet naturel et donnent au dispositif un aspect apaisant.

De préférence, le boîtier 204 est constitué d'un matériau hydrophile. Par exemple, le matériau du boîtier 204 peut être : une céramique, un polyamide (PA), un silicone ou un polymère hydrophile. La surface extérieure du boîtier 204 peut également être traitée avec un traitement de surface hydrophile, par exemple acuWet^{®} d'Aculon, un polymère hydrophile, ou Pebax^{®} d'Arkema.

### Ensemble de test

La station 200 comprend un orifice de collecte 218, situé par exemple sur la coque arrière 208. L'orifice de collecte 218 est configuré pour collecter l'urine s'écoulant sur la surface du boîtier 204. La station 200 peut comprendre également un orifice de drainage configurée pour drainer le liquide hors du dispositif 100.

Un ensemble de test 230 est disposé à l'intérieur du boîtier 204 et configuré pour effectuer une analyse sur l'urine collectée par l'orifice de collecte 218. La station 200 comprend un compartiment de réception 212, situé à l'intérieur du boîtier 204. Le compartiment de réception 212 est configuré pour recevoir au moins partiellement la cartouche 202.

Dans un mode de réalisation, la cartouche 202 peut être montée de manière rotative autour de l'axe de rotation X, une fois en position dans le compartiment annulaire 212. Le compartiment de réception 212 est alors un compartiment annulaire. Le compartiment de réception 212 s'étend typiquement sur 360° et forme une gorge configurée pour recevoir au moins partiellement la cartouche 202.

En variante, la cartouche 202 peut être insérée en translation dans la station 200. Le compartiment de réception 212 forme alors un canal longitudinal pour recevoir au moins partiellement la cartouche 202.

D'autres formes de compartiment de réception 212 et de manière d'insérer la cartouche 202 peuvent être évidemment envisagées.

L'ensemble de test 230 peut comprendre une pompe, un injecteur et un analyseur. La pompe aspire l'urine de l'orifice de collecte 218, puis l'injecteur injecte l'urine dans au moins une chambre de la cartouche et l'analyseur analyse l'urine. Dans un mode de réalisation, chaque chambre loge au moins un réactif et l'analyseur obtient certaines valeurs de propriétés (par exemple, des propriétés physiques/chimiques, telles que la couleur) des réactifs après qu'ils sont entrés en contact avec l'urine. Dans un cas, l'analyseur est un analyseur optique configuré pour analyser les propriétés optiques du réactif. En variante ou en complément, l'analyseur effectue une analyse optique ou spectroscopique directement de l'urine pour en déterminer certaines propriétés. L'injecteur et la cartouche peuvent se déplacer l'un par rapport à l'autre afin que l'injecteur puisse ouvrir (par exemple, percer) la chambre, par exemple à l'aide d'une aiguille ou d'un dispositif semblable à une aiguille. Le fonctionnement du dispositif 100 sera décrit plus en détail par la suite.

Les dimensions relatives à la cartouche 202 sont divulguées dans les documents WO'909, WO'933 et WO'80X. La dimension maximale du dispositif 100 transversalement à l'axe de rotation X est inférieure à 15 cm, voire inférieure à 10 cm. La dimension maximale du dispositif le long de l'axe de rotation X est inférieure à 5 cm.

### Cartouche

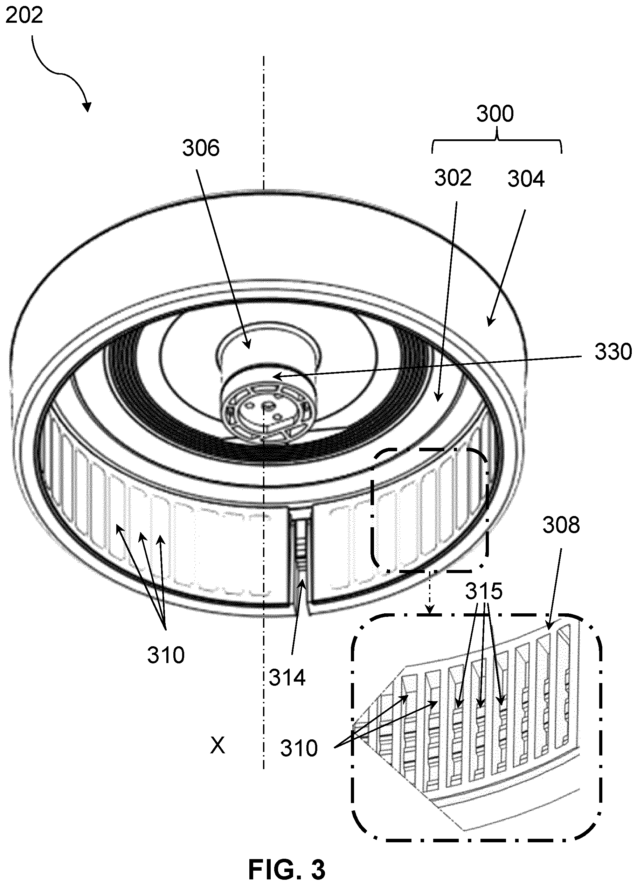

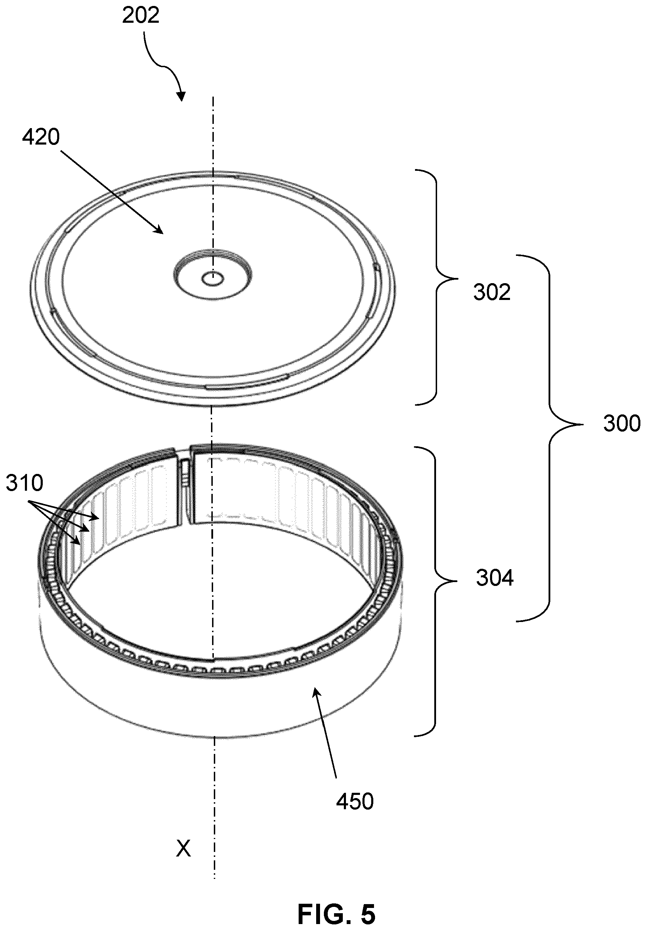

Les figures 3 à 5, 7 et 8 représentent différents modes de réalisation de la cartouche 202. La cartouche 202 comprend un support 300 et une pluralité de chambres 310.

La cartouche 202 peut comprendre au moins dix chambres 310, notamment au moins vingt-cinq chambres 310, voire au moins cinquante chambres 310.

Chaque chambre 310 est propre à recevoir de l'urine pour analyse d'urine. Dans un mode de réalisation, chaque chambre 310 loge un réactif 315 configuré pour réagir de manière spécifique au contact de l'urine de l'utilisateur. En variante ou en complément, l'urine injectée dans la chambre est analysée directement, par exemple par analyse optique ou spectroscopique. Chaque chambre 310 est initialement étanche. Par "chambre étanche", on entend une chambre étanche à l'humidité extérieure à la chambre. Ainsi, chaque chambre 310, et éventuellement le réactif 315 associé, sont séparés fluidiquement du reste de la cartouche 202. Le réactif est par exemple disposé sur une bandelette de test. Chaque chambre 310 comprend alors une ou plusieurs bandelettes de test. En variante, le réactif 315 peut être un réactif liquide reçu dans la chambre respective.

Une chambre 310 est notamment formée par des parois, qui peuvent être en un ou plusieurs matériaux. Le document EP4338839 décrit des modes de réalisation des chambres 310. Les chambres 310 étanches peuvent notamment être formées à l'aide d'une superposition de couche en matériaux différents.

Chaque chambre 310 étanche comprend un opercule 610 perçable, par exemple par une aiguille , afin de permettre l'injection de l'urine par l'injecteur de l'ensemble de test 230. Dans un mode de réalisation, l'opercule 610 est transparent ou translucide pour permettre le passage de la lumière à l'intérieur de la chambre et/ou pour effectuer l'analyse optique, et ainsi permettre l'analyse du résultat de la réaction du réactif avec l'urine ou l'analyse optique directe. Une fois percée, la chambre 310 perd son étanchéité et est alors en communication fluidique avec l'extérieur et donc avec l'intérieur de la station.

La pluralité de chambres 310 est maintenue en position par le support 300. Autrement dit, le support 300 maintient mécaniquement les chambres 310 à leur position entre elles lors de la manipulation de la cartouche 202 pendant son insertion ou désinsertion de la station 200 par un utilisateur, ainsi que durant la coopération entre la station 200 et la cartouche 202 lors du fonctionnent du dispositif 100. Le support 300 est donc l'élément structurel permettant d'assurer l'intégrité physique de la cartouche 202. Le support 300 supporte donc les chambres 310 et permet la coopération mécanique avec la station 200. Dans le cadre d'une utilisation normale de la cartouche 202 et du dispositif 100, les chambres 310 sont fixées au support 300 et ne se déplacent pas par rapport au support 300.

Le support 300 est notamment rigide. Par "rigide", il est entendu que le support résiste à des contraintes exercées habituellement par un utilisateur et par la station lors d'une utilisation normale du dispositif. Le support 300 peut toutefois présenter une certaine flexibilité, notamment une flexibilité inhérente aux matériaux mais ne présentant pas d'utilité fonctionnelle.

Dans un mode de réalisation, une portion du support 300, notamment une portion du support 300 adjacente à une chambre 310, est transparente ou translucide, pour permettre le passage de la lumière à l'intérieur de la chambre 310 et/ou pour effectuer l'analyse optique dans la chambre 310, et ainsi permettre l'analyse du résultat de la réaction du réactif avec l'urine ou l'analyse optique directe.

Au moins une portion du support 300, appelée portion dessiccante 420, est composée d'un dessiccant solide. Le dessiccant solide est apte à absorber l'humidité de l'air au contact de la portion dessiccante 420. En particulier, la portion dessiccante 420 possède une capacité d'absorption de l'eau comprise entre 100 mg/g et 300 mg/g, notamment entre 140 mg/g et 200 mg/g. Le dessiccant solide comprend en particulier un matériau hygroscopique, autrement dit un matériau qui a la capacité d'absorber l'humidité de l'air. Autrement dit, le dessiccant solide est configuré pour absorber des molécules de vapeur d'eau au contact de la portion dessiccante 420.

Dans un mode de réalisation, le dessiccant solide est un mélange d'au moins deux matériaux, en particulier un mélange d'un polymère plastique et d'un matériau dessiccant. Le matériau dessiccant peut se présenter par exemple sous forme de poudre diluée dans le polymère plastique. Le dessiccant solide est par exemple formé de polypropylène chargé en zéolite.

La portion dessiccante 420 se trouve à l'extérieur des chambres 310 étanches. Autrement dit, il n'y a pas de communication fluidique entre le volume interne des chambres 310 étanches et la portion dessiccante 420. Plus précisément, il n'y a donc pas de contact entre la portion dessiccante 420 et le volume interne des chambres 310 lorsque les chambres 310 sont fermées par l'opercule 610.

La portion dessiccante 420 n'a donc pas une fonction dessiccante dans les chambres 310 étanches pour préserver l'intégrité des réactifs avant leur utilisation dans la station 200. A cet effet, la cartouche 202 peut comprendre en outre un dessiccant additionnel disposé dans au moins une chambre 310 étanche. Avantageusement, un dessiccant additionnel est disposé dans chaque chambre 310 étanche. Le dessiccant additionnel est donc différent de la portion dessiccante 420. Le dessiccant additionnel est par exemple une bande de matériau dessiccant. En variante ou en complément, le dessiccant additionnel est par exemple au moins une partie d'une paroi de la chambre 310 formée d'un matériau dessiccant.

La portion dessiccante 420 peut représenter au moins 25%, préférentiellement 50%, du support 300 en surface. Autrement dit, au moins 25%, préférentiellement 50%, de la surface extérieure du support 300 est composée du dessiccant solide. Ainsi, la portion dessiccante 420 représente une partie significative du support 300 et offre donc un pouvoir dessiccant important sans occuper de place supplémentaire.

Le support 300 présente ainsi donc une double fonction. Le support 300 permet de supporter et maintenir en position les chambres 310 et donc les réactifs 315 dans la cartouche 202, tout en participant à la réduction de l'humidité dans la station 200. En effet, comme expliqué plus haut, lorsque la cartouche 202 est insérée dans la station 200 et que les chambres 310 sont percées, l'urine injecté dans la chambre 310peut par la suite s'évaporer et s'échapper hors de la chambre 310. La portion dessiccante 420 est alors apte à absorber au moins une partie de cette humidité additionnelle. La portion dessiccante 420 permet donc d'éviter que les injections répétées d'urine dans les chambres augmentent l'humidité dans le dispositif d'analyse d'urine de manière trop importante et préserve ainsi le bon fonctionnement des dispositifs électroniques de la station 200.

Comme cela sera décrit par la suite, la portion dessiccante 420 peut être une pièce du support 300 ou bien une partie d'une pièce du support 300.

Dans un mode de réalisation, la portion dessiccante 420 forme un socle sur lequel sont montées les chambres 310. Ainsi, les chambres 310 reposent sur le socle. Autrement dit, au moins une face des chambres 310 étanches est en contact avec le socle, soit directement, soit via une portion de réception.

En variante ou en complément, la portion dessiccante 420 comprend une zone de réception d'effort 306 d'un actionneur 330 de la station 200. La zone de réception d'effort 306 est une zone de coopération mécanique entre la cartouche 202 et la station 200, par exemple pour mettre la cartouche 202 en rotation et/ou en translation par rapport à la station 200 lors du fonctionnement du dispositif 100, notamment pour faire défiler les chambres 310 devant l'injecteur et/ou l'analyseur de l'ensemble de test 230. La portion dessiccante 420 permet donc à la fois de réduire l'humidité dans la station 200 et de faire la liaison mécanique entre la cartouche 202 et la station 200. La zone de réception d'effort 306 peut être un moyeu apte à recevoir un arbre mobile en rotation.

Dans un mode de réalisation, au moins une portion du support 300, appelée portion transparente 450, est composée d'un matériau transparent ou translucide. La portion transparente 450 est par exemple formée d'un matériau plastique. La portion transparente 450 peut former au moins une partie des chambres 310 pour permettre le passage de la lumière et l'analyse des réactions.

Dans un mode de réalisation, la portion dessiccante 420 et la portion transparente 450 forme deux pièces distinctes du support 300, assemblées entre elle. La portion dessiccante 420 et la portion transparente 450 sont par exemple collées ou clipsées entre elles. Dans un mode de réalisation, la portion dessiccante 420 et la portion transparente 450 forme entièrement le support 300.

### Support rotatif

En référence aux figures 3 à 6, le support 300 est par exemple un support rotatif configuré pour être entraîné en rotation par la station 200.

Dans un mode de réalisation, le support rotatif 300 présente une forme de cylindre circulaire droit d'au moins 80% d'une forme de cylindre creux s'étendant annulairement autour d'un axe qui est, lorsque la cartouche 202 est montée dans la station 200, l'axe de rotation X.

Comme visible sur la figure 2, le compartiment de réception 212 de la station 200 présente alors une forme de compartiment annulaire.

Le support 300 rotatif peut comprendre une partie annulaire 302 et une partie cylindrique 304, qui s'étend à partir d'une extrémité radiale extérieure de la partie annulaire 302. Lorsque la cartouche 202 est insérée dans la station 200, la partie cylindrique 304 est logée à l'intérieur du compartiment annulaire 212.

Dans un mode de réalisation, la partie annulaire 302 forme la portion dessiccante 420. La portion dessiccante 420 présente sensiblement une forme de disque. Cette forme permet d'optimiser le rapport entre la surface de contact avec l'air et le volume occupé par le disque. Le disque présente par exemple un diamètre compris entre 3 cm et 10 cm. Le disque présente une épaisseur comprise entre 1 mm et 10 mm. La portion dessiccante 420 peut former le socle.

Dans ce mode de réalisation, la partie cylindrique 304 peut former la portion transparente 450. En variante, la partie cylindrique 304 peut être en matériau opaque.

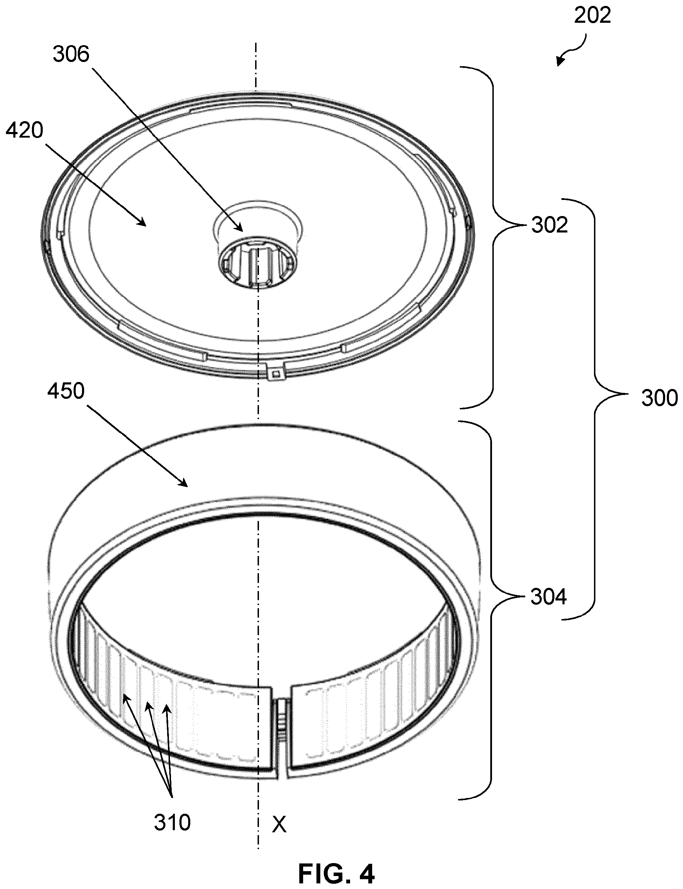

Comme visible sur les figures 3 et 4, chaque chambre 310 peut s'étendre selon une direction principale orthogonale au socle. Les chambres 310 sont positionnées le long de la portion cylindrique 304, de manière à pouvoir défiler sélectivement et/ou successivement devant l'injecteur et l'analyseur. La portion cylindrique 304 fait alors office de portion de réception. Les chambres 310 sont disposées les unes à côté des autres en forme de cylindre circulaire droit d'au moins 80 % du cercle. Les chambres 310 sont toutes à égale distance de l'axe de rotation X, de sorte que l'injecteur peut injecter sélectivement l'urine une fois que la chambre 310 étanche souhaitée est positionnée à l'endroit voulu face à l'injecteur. L'injecteur peut se déplacer vers la chambre 310 étanche et percer l'opercule 610 fermant la chambre 310 étanche. Un orifice de vidange 314 est prévue dans le support rotatif 300 pour permettre l'évacuation de l'urine de l'injecteur vers l'extérieur du dispositif 100, via l'orifice de drainage 314 disposée sur le boîtier 204.

Dans l'exemple illustré, la partie annulaire 302 du support rotatif 300 reste à l'extérieur du compartiment annulaire 212 pour renforcer la partie cylindrique et/ou entraîner en rotation la cartouche 202. À cette fin, la partie annulaire 302 peut comprendre la zone de réception d'effort 306, par exemple sous la forme d'un accouplement mécanique (ici un moyeu) qui coopère avec l'actionneur 330 de la station (ici un arbre). La zone de réception d'effort 306 se présente par exemple sous la forme d'un arbre logé dans une cavité 250 de la station 200.

### Variantes de support

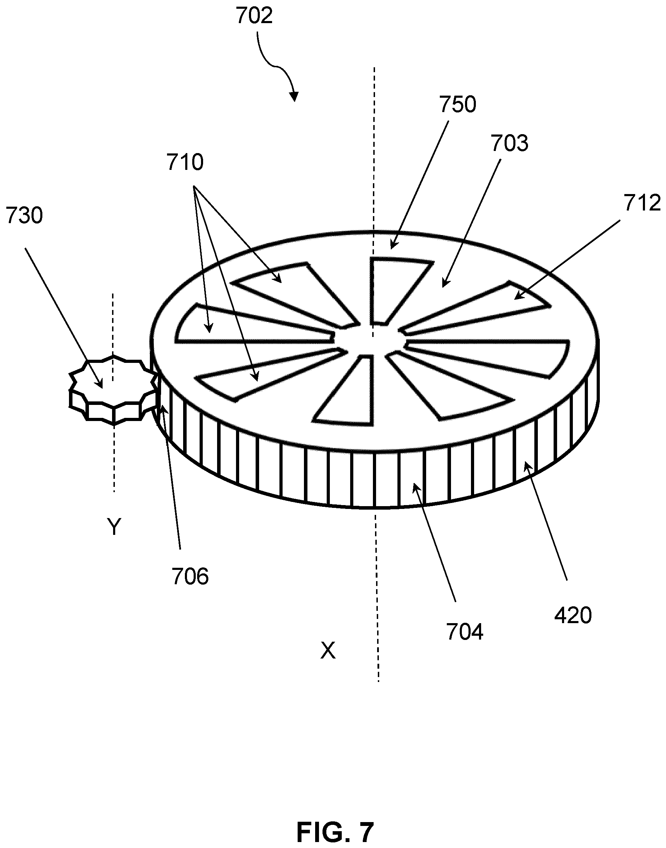

En variante, en référence à la figure 7, la cartouche 702 présente une forme cylindrique pleine. Le compartiment de réception 212 de la station 200 présente alors une forme complémentaire du cylindre. Le support rotatif 300 peut comprendre une partie annulaire 703 et une partie cylindrique 704, qui s'étend radialement à l'intérieur de la partie annulaire 703. Chaque chambre 710 étanche peut s'étendre orthogonalement à la direction de rotation X. Chaque chambre 710 peut ainsi s'étendre radialement, entre la partie cylindrique 704 et le centre de la partie annulaire 703. La portion dessiccante 420 est formée par la partie cylindrique 704, telle que décrite précédemment. La partie annulaire 703 peut former la portion transparente 750. Chaque chambre 710 est refermée par un opercule 712 disposé par-dessus la partie annulaire 703. En fonctionnement, l'analyseur et l'injecteur sont disposés au-dessus et/ou en dessous selon l'axe de rotation X de la cartouche 202. La portion dessiccante 420 comprend ici une zone de réception d'effort 706 sous la forme de dentures actionnable par un actionneur 730 sous la forme d'une roue d'engrenage. La rotation de l'actionneur selon un axe Y parallèle à l'axe X entraine la rotation de la cartouche 702 pour faire défiler les chambres 710 devant l'injecteur et/ou l'analyseur.

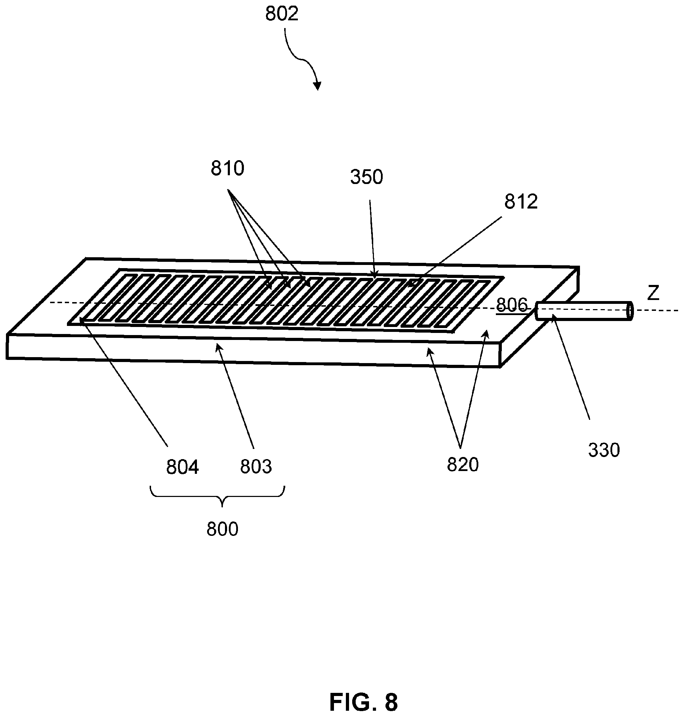

En variante, en référence à la figure 8, la cartouche 802 présente une forme de parallélépipède s'étendant principalement selon une direction principale Z. Le compartiment de réception 212 de la station 200 présente alors une forme de canal, complémentaire de la cartouche 802. Le support 800 peut comprendre un socle 803 et, dans un mode de réalisation, une portion de réception 804, apte à recevoir les chambres 810 étanches. Chaque chambre 810 peut s'étendre orthogonalement à la direction principale Z. Les chambres 810 s'étendent parallèlement entre elles. La portion dessiccante 820 est formée par le socle 803. Chaque chambre 810 est refermée par un opercule 812 disposé. L'opercule 812 et/ou la portion de réception 804 peuvent être transparent. En fonctionnement, l'analyseur et l'injecteur sont disposés au-dessus et/ou en dessous des chambre 810, selon une direction orthogonale à la direction principale Z. La portion dessiccante 820 comprend ici une zone de réception d'effort 806 sous la forme d'une paroi apte à être poussée ou tirée par un actionneur 330 sous la forme d'une tige s'étendant selon la direction principale Z et par exemple actionnée par un moteur pas à pas. La translation de l'actionneur 330 selon la direction principale Z entraine la translation de la cartouche 802 pour faire défiler les chambres 810 devant l'injecteur et/ou l'analyseur.

### Interaction station-cartouche

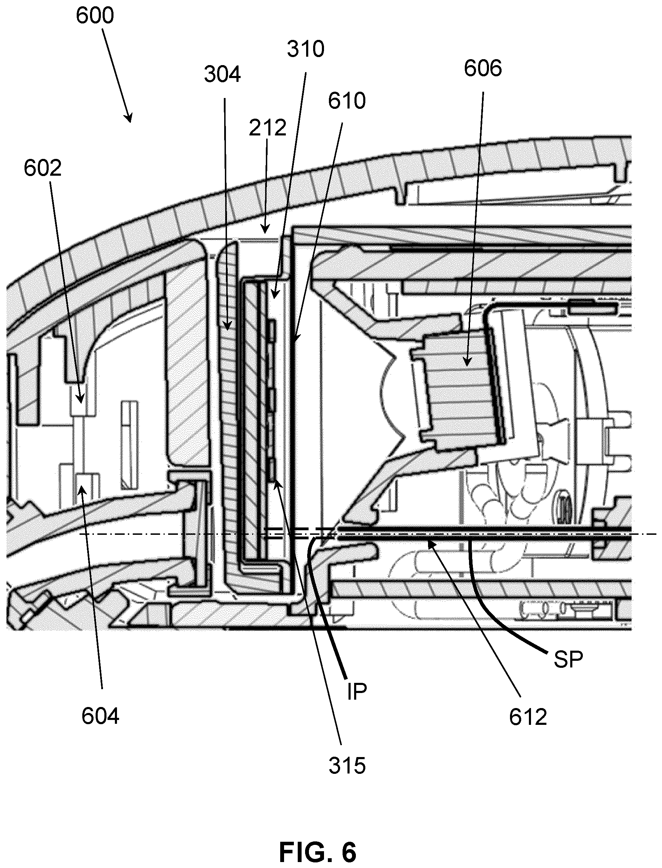

La figure 6 montre plus en détail l'interaction entre la cartouche 202 selon le mode de réalisation des figures 3 à 5 et la station 200 lorsque ou après l'activation de l'injecteur. L'analyseur 600 comprend au moins une source lumineuse 602, 604 (par exemple, deux sources lumineuses ; notamment quatre sources lumineuses) et au moins un capteur optique 606. La lumière va de la source lumineuse 602, 604 au capteur optique 606 en traversant la cartouche 202 et en particulier la partie cylindrique 304 et éventuellement le réactif 315.

Dans un mode de réalisation, l'analyseur 600 est configuré pour mesurer l'absorbance d'une partie des réactifs 315.

L'absorbance est détectée par la source lumineuse (par exemple une LED) qui peut faire passer de la lumière à travers la bande, et le capteur optique qui reçoit le spectre avec une dizaine de longueurs d'onde.

Dans une variante, le capteur de lumière est une caméra capable de détecter un changement de couleur, notamment un changement d'intensité de couleur, d'une partie des réactifs 315.

La caméra peut détecter une couleur en valeurs RVB par exemple.

L'injecteur comprend une extrémité d'injection 612 (par exemple une aiguille), qui peut être déplacée entre une position d'attente SP et une position d'injection IP. Dans la position d'attente SP, l'extrémité d'injection 612 est à l'extérieur de la cartouche 202 (dans une position la plus intérieure radialement), de sorte que la cartouche 202 peut tourner librement dans le compartiment annulaire 212. Dans une position d'injection IP, l'extrémité d'injection 612 a percé l'opercule 610 pour accéder à l'intérieur de la chambre 310 et peut injecter un peu d'urine sur le réactif 315.

En position SP, l'injecteur est situé radialement à l'intérieur de la chambre annulaire. Ceci permet de maximiser le rayon du compartiment annulaire tout en minimisant la taille de la station 200.

Dans une variante de l'ensemble de test 230 divulgué ci-dessus, l'homme de métier comprendra que chaque ensemble de test configuré pour analyser l'échantillon d'urine collecté par l'orifice de collecte 218 peut être disposé à l'intérieur du boîtier 204.

## Revendications

1. Cartouche (202) pour analyse d'urine comprenant :
- un support (300),
- une pluralité de chambres (310) étanches, chaque chambre (310) étanche étant propre à recevoir de l'urine pour analyse d'urine,
dans laquelle :
- la pluralité de chambres (310) étanches est maintenue en position par le support (300),
- au moins une portion du support, appelé portion dessiccante (420), est composée d'un dessiccant solide, apte à absorber l'humidité de l'air au contact de la portion dessiccante (420), la portion dessiccante (420) étant à l'extérieur des chambres (310) étanches.

2. Cartouche (202) selon la revendication 1, dans laquelle le dessiccant solide est un matériau hygroscopique.

3. Cartouche (202) selon la revendication 1 ou 2, dans laquelle le dessiccant solide est un mélange bi-matériaux formé d'un polymère plastique et d'un matériau dessiccant.

4. Cartouche (202) selon l'une quelconque des revendications précédentes, dans laquelle la portion dessiccante possède une capacité d'absorption comprise entre 100 mg/g et 300 mg/g, notamment entre 140 mg/g et 200 mg/g.

5. Cartouche (202) selon l'une quelconque des revendications précédentes, dans laquelle la portion dessiccante (420) représente au moins 25%, préférentiellement 50%, du support (300) en surface.

6. Cartouche (202) selon l'une quelconque des revendications précédentes, dans laquelle la portion dessiccante (420) est rigide.

7. Cartouche (202) selon l'une quelconque des revendications précédentes, dans laquelle la portion dessiccante (420) comprend une zone de réception d'effort (310) d'un actionneur (330) d'une station (200).

8. Cartouche (202) selon l'une quelconque des revendications précédentes, dans laquelle la portion dessiccante (420) forme un socle sur lequel sont montées les chambres (310).

9. Cartouche (202) selon l'une quelconque des revendications précédentes, dans laquelle chaque chambre (310) comprend un opercule (610) perçable, par exemple par une seringue.

10. Cartouche (202) selon l'une quelconque des revendications précédentes, dans laquelle la cartouche (202) comprend en outre un dessiccant additionnel, différent de la portion dessiccante (420), disposé dans au moins une chambre (310).

11. Dispositif (100) d'analyse de l'urine comprenant :
- une cartouche (202) selon l'une quelconque des revendications précédentes,
- une station (200) configurée pour être positionnée sur une paroi (112) d'une cuvette de toilettes (106), la station (200) comprenant :
+ un boitier (204) comprenant un compartiment de réception (212) dans lequel la cartouche (202) est au moins partiellement reçue,
+ un injecteur, configuré pour injecter du fluide dans l'une des chambres (310),
+ un analyseur pour analyser l'urine injectée dans ladite chambre (310).

12. Dispositif (100) selon la revendication 11, dans lequel la cartouche (202) est agencée de manière amovible dans le boitier (204).
